# EUROPEAN PATENT SPECIFICATION

(11) **EP 4 322 965 B1**
(45) Date of publication and mention of the grant of the patent: **25.03.2026**
(21) Application number: 22701582.3
(22) Date of filing: 20.01.2022
(51) Int. Cl.: A61K 47/36

(54) **COMPOSITION COMPRISING HIGH MOLECULAR WEIGHT HYALURONIC ACID AND AN OLIGOMER OF HYALURONIC ACID**
ZUSAMMENSETZUNG MIT HYALURONSÄURE MIT HOHEM MOLEKULARGEWICHT UND EINEM OLIGOMER AUS HYALURONSÄURE
COMPOSITION COMPRENANT DE L'ACIDE HYALURONIQUE DE POIDS MOLÉCULAIRE ÉLEVÉ ET UN OLIGOMÈRE D'ACIDE HYALURONIQUE

(30) Priority: 14.04.2021 IT 202100009425
(43) Date of publication of application: 21.02.2024
(73) Proprietor: Lem Compounding Research ITA Srl, 20823 Lentate sul Seveso (MB) (IT)
(72) Inventor: CASTIGLIONI, Mauro, 20823 Lentate sul Seveso (MB) (IT); CASTIGLIONI, Francesca, 20823 Lentate sul Seveso (MB) (IT); TORRETTA, Beatrice, Legnano MI (IT)
(74) Representative: Serravalle, Marco
(86) International application number: PCT/EP2022/051279
(87) International publication number: WO 2022/218578

(56) References cited:
- CN-A- 107 007 876
- US-A1- 2008 057 091
- US-A1- 2010 323 983

## Description

### Filed of the invention

**.** The present invention is directed to a process for the preparation of a composition of hyaluronic acid having high anti-inflammatory properties.

### Background of the invention

**.** Hyaluronic acid (HA) was first isolated in 1934, and its chemical structure was defined in 1954. It is a sulfated and linear polysaccharide belonging to the family of glycosaminoglycans (GAG). Its primary structure is represented by disaccharide repetitive units of N-acetyl-D-glucosamine and D-glucuronic acid. Unlike all other GAGs, which have a molecular weight between 10 and 100 kDa, the molecular weight of HA is in the order of 3,000-6,000 kDa. It is the GAG present in greater quantity in mammals, but unlike most GAGs, it cannot be efficiently isolated from the body of mammals, since during extraction HA undergoes degradation processes that significantly reduce its molecular weight.

**.** Currently, commercial HA is obtained by microbial production. The first industrialscale microbial production was obtained in 1980 by Shiseido. The most commonly used strain in the production of HA is S. zooepidemicus, which can produce 6~7 g/l of HA under suitable culture conditions. HA obtained from microbial production has a structure and a molecular weight similar to HA endogenously produced by humans. However, for many applications, it is preferable to use a somewhat lower molecular weight and HA is subjected to depolymerization processes.

**.** The most common depolymerisation process is enzymatic depolymerisation using hyaluronidase. The molecular weight of depolymerized HA may vary between 2,000 kDa and 10 kDa depending on process conditions, but the dispersion index of depolymerized HA is always broad.

**.** WO 2014/159592 discloses a process for the depolymerization of HA using ultrasound. By depolymerising high molecular weight HA at 20-80 kHz for 3-6 hours, it is possible to obtain HA with an average molecular weight between 100 kDa and 500 kDa.

**.** US 2010/323983 discloses two separate compositions, the first comprising HA having Mw comprised between 100 and 10,000 kDa, and the second comprising a dimer of NAC glucuronic acid, which is different from the repeating unit of HA. The two solutions are administered separately.

**.** CN 107 007 876 discloses a hyaluronic acid medical sterile dressing comprising the following components: 0.01% to 1% high molecular weight HA, 0.01 to 1 % small molecular weight HA, 0.01% to 1% oligomeric HA. The molecular weight of the high molecular weight HA is 1200kDa to 2000kDa; the molecular weight of the small molecular weight HA is 100KDa to 1000KDa; the molecular weight of the oligomeric hyaluronic acid or salt thereof is 1000Da to 10KDa.

### Summary of the invention

**.** The invention is directed to a process for the preparation of a HA composition having high anti-inflammatory properties, the process comprising the following steps:
a. preparing a solution of HA having molecular weight Mw comprised between 500 kDa and 6,000 kDa at a concentration comprised between 0.1 g/L and 5 g/L;
b. maintaining the temperature of the solution lower than or equal to 10°C;
c. submitting the solution to pulse sonication for a time comprised between 1' and 30',
wherein the pulse of pulse sonication is comprised between 5" and 15" for both the sonication phase and the pause between two sonications, and the sonication power per mass unit of solution is from 200 to 2000 kW/kg.

### Detailed description of the invention

**.** Pulsed sonication is performed by subjecting an HA solution to sonication for a set period of time and suspending sonication for another set period of time. Preferably the time period is between 5s and 15s both for the sonication passage and for the pause between two sonication passages. For example, sonication can be performed for 10 seconds and then suspended for 10 seconds. The break helps to keep the temperature below the set temperature. The sonication period may be varied as long as the temperature is kept below the set value. Sonication can be performed at various powers. Preferably, sonication is performed at a power per mass unit comprised between 200 and 2000 W/kg of solution. The sonication frequency can be varied in a wide range. The preferred frequency is 20 kHz. The level of amplification can be varied in a wide range and is preferably between 50% and 100%. The sonication time is preferably between 1' and 30', more preferably between 2' and 20', even more preferably between 5' and 15'.

**.** HA obtained by the above defined process is characterized by a prevalent fraction of HA having average molecular weight lower than the starting HA. For example, the average molecular weight Mw after sonication is about 30% to 70% of the starting molecular weight. Then, starting from a molecular weight Mw of about 2000 kDa, we will have after sonication a prevalent fraction of HA having molecular weight between 600 kDa and 1,400 kDa. Furthermore, it was observed that in addition to the main fraction with a lower average molecular weight than the original HA, an oligomeric fraction with a weight average molecular weight Mw lower than 2,000 Da is present in the sonicated HA. Sonication, however, also forms a small oligomeric fraction with a molecular weight of less than 2,000 Da in quantities ranging from 0,005 % by weight to 1,0 % by weight.

**.** The sonicated composition as defined above is preferably mixed with at least one high Mw molecular weight HA. High molecular weight means an HA having molecular weight Mw comprised between 500 kDa and 6,000 kDa, preferably between 1,000 kDa and 4,000 kDa, even more preferably between 1,500 kDa and 3,000 kDa.

**.** In the final composition, the weight ratio between sonicated HA and the sum of sonicated HA and high Mw HA is between 5 % and 100 %, preferably between 10 % by weight and 50 % by weight, even more preferably between 15 % by weight and 30 % by weight.

**.** It has been surprisingly found that an HA composition obtained by a process as defined above, is a composition having high anti-inflammatory activity.

**.** Without being bound by the theory, it is believed that the presence of even a small fraction of oligomers of HA having average molecular weight Mn below 2000 Da can improve the penetration of high molecular weight HA in topical applications.

**.** Although the tests were conducted to detect the anti-inflammatory activity of the composition in comparison with the known high-weight HA known in the art, it is believed that the composition according to the invention can have very high activities even in a field where HA is widely used, namely anti-age and more generally the aesthetic sector.

### Experimental part

**.** Two commercially available HA have been used for the tests. The first, used in comparative example 1, has Mw of 2,142,579 Da, the second, used in comparative example 2, has Mw of 1,731,674.

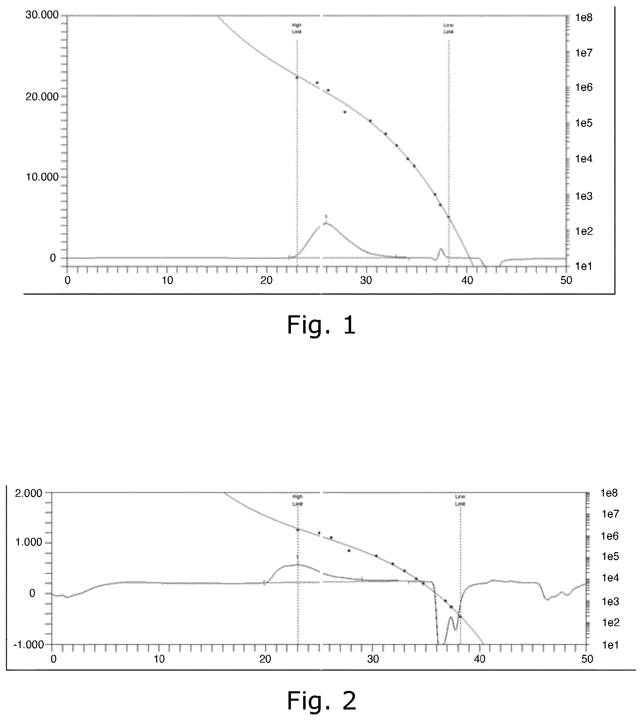

**.** 1 g sample of the first HA with higher molecular weight was dissolved in water and the solution brought to 100 ml. The solution was cooled down to 10°C and subjected to pulsed sonication for 10' with a pulse interval of 10", a frequency of 20 MHz, a power of 20W and 100% amplification. Figure 1 shows the graph of the Gel Permeation Chromatography (GPC) of this composition. Sonicated HA showed Mw 711,028, Mn 412,925 and PD 1.7219. The x axis reports the retention time in minutes, while the y axis reports on the left side the response in mV (linear scale), and on the right side the Mw in Dalton (exponential scale).

### Quantification of the amount of oligomer in sonicated HA

**.** The resulting composition has been diluted in a 5:1 ratio and subjected to GPC with an Agilent Technologies^{®} 1260 Infinity (serial # DEAB904745) equipped with a guard column (Part # CHO-9225, size 35 x 7.8 mm, S/N H18-355697) and three columns installed in series: Polysep-GFC-P 6000 (Part # CHO-9231, size 300 x 7.8 mm, S/N H18-3556687s separation range 100K-15M, pH range 3.0-12.0), Polysep-GFC-P 5000 Part # CHO-9230, size 300 x 7.8 mm, S/N H19-037701, separation range 50K-2M, pH range 3.0-12.0) and Polysep-GFC-P 4000 (Part # CHO-9229, size 300 x 7.8 mm, S/N H19-355775, separation range 3K-400K, pH range 3.0-12.0). All columns are columns PolySep^{™}, by Phenomenex^{®} (Phenomenex S.r.l., Castel Maggiore (BO), Italy).

**.** Chromatographic conditions - The mobile phase was prepared by dissolving sodium sulfate (Na2so4) at 0.005 M concentration, pH 7.4 in ultrapure H₂O (milliO system) at RT, the pH was corrected to 7.4 using NaOH 0.1M. Flow rate 0.8 ml/min. Injected volume 20 µl. Quantification of the peak of the oligomer was done with a standard of PEG 1000. The analysis was made on three separate samples and gave the following oligomer values per 100 mg sample: 78,6 µg, 84,55 µg, 67,20 µg, corresponding to a concentration of 0,078 %, 0,085% and 0,067%.

**.** The composition of example 3 was obtained by mixing 60% HA of comparative example 2, 20% of HA of comparison example 1 and 20% of HA sonicated as above.

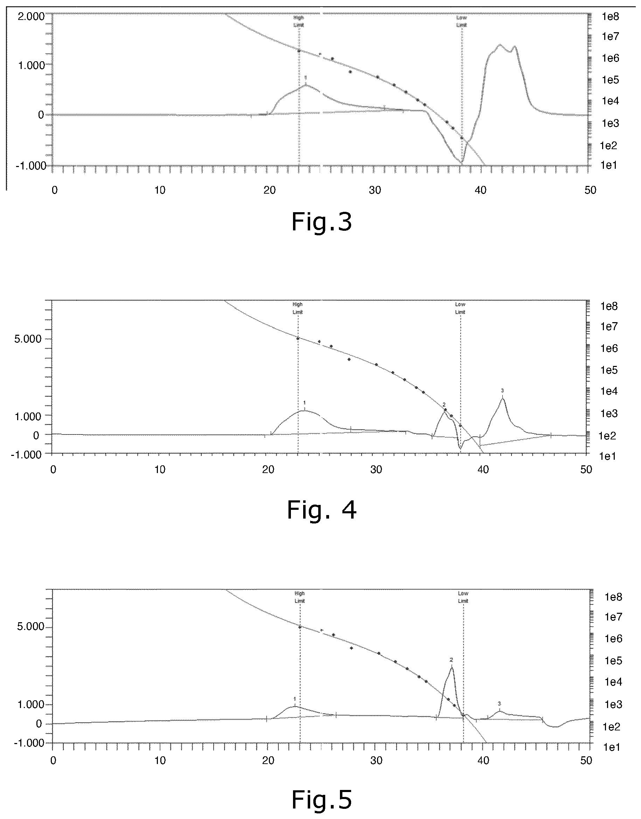

**.** The composition of example 4 was obtained by mixing 60% HA of comparative example 2 with 40% sonicated HA.
20LC26P1 Comparative example 1: a single peak Mw = 2.142.579 Da, Pd = 1,76 (Fig. 2). 20LC26P2 Comparative example 2: a single peak Mw = 1.731.674 Da, Pd = 2,13 (Fig. 3). 20LC6P1 Example 3: 1^{st} peak Mw = 1.807.836 Da, Pd = 1.45, 2^{nd} peak Mw = 1050 (Fig. 4). 20LC6P4 Example 4: 1^{st} peak Mw = 2.390.142, Pd = 1.25, 2^{nd} peak Mw = 917 (Fig. 5). In the figures, the x axis reports the retention time in minutes, while the y axis reports on the left side the response in mV (linear scale), and on the right side the Mw in Dalton (exponential scale).

### Evaluation of anti-inflammatory properties

**.** Cytokines are small proteins (5 to 20 kDa) known to play an important role in the immune system; in particular, they are able to influence the activation, maturation, growth and differentiation of several cell populations. Amongst the wide family of cytokines, one can be particularly identified as the most direct and reliable inflammation marker: IL-8 (Harada et al., 1994).

**.** IL-8 is a member of the chemokine family C-X-C (the acronym is related to the structural sequence cysteine-amino acid-cysteine in the amino terminal portion of the cytokine); it is not produced directly by keratinocytes, but its secretion is promoted in an inflamed environment in response to other cytokines previously involved in the acute phase response (IL-1a, IL-1b, TNF-a, INF-g) (Wilmer et al., 1995). Its pivotal function is to play a role as a chemoattractant, mainly against polymorphonuclear and lymphocyte neutrophils (Larsen et al., 1989), but also towards basophiles, inducing histamine secretion in these cells (Wilmer et al., 1995). Specifically, it appears to be involved in the adhesion of leukocytes to endothelial cells and diapedesis. In addition, it could also exert an effect in activating immune cells and in promoting angiogenesis (Moore et al., 1998); it causes granule exocytosis from neutrophils and increases cytosolic free calcium, resulting in a remodeling in neutrophil morphology that accompanies surface membrane remodeling and increased expression of adhesion molecules, such as Cd11b/CD18 (complement receptor type 3) Cd11c/CD18 and complement receptor type I (Baggiolini et al., 1992).

**.** IL-8 is produced as a precursor of 99 amino acids which is then cleaved into several variants of which the most represented form is composed of 72 amino acids. The production of IL-8 has been demonstrated in a wide variety of cells, including monocytes, T-cells, neutrophils, vascular endothelial cells, dermal fibroblasts, hepatocytes, chondrocytes, synovial cells and keratinocytes (Harada et al., 1994). Its secretion is particularly active in chronic secretion conditions, such as rheumatoid arthritis, inflammatory bowel diseases, and, for the skin, diseases such as psoriasis (Schroder et al., 1992) or palmoplantar pustulosis (Ozawa et al., 2005), where the expression of its mRNA has been identified as prominent in keratinocytes and its detection has been explored as a therapeutic option.

**.** Thus, the measurement of this cytokine in an in vitro model composed of keratinocytes, is a useful and sensitive tool to measure the anti-inflammatory effect of a cosmetic product, including raw materials.

**.** The purpose of the test is to evaluate the anti-inflammatory potential of the product tested by measuring the levels of IL-8 after stimulation of human keratinocytes with an inflammatory stimulus, LPS. Preliminary experiments (MTT tests) were also conducted to identify non-cytotoxic product concentrations.

**.** The keratinocytes used in the experiments were a line of human cells (Hacat, BS code CL 168), supplied by I.Z.L.E.R. (Istituto Zooprofilattico della Lombardia e Emilia-Romagna). The cell line was grown in complete sterility and incubated at 37 °C in a 5% carbon dioxide atmosphere.

**.** The MTT test is a colorimetric cytotoxicity test used to measure cell proliferation and viability based on mitochondria efficiency. MTT (3-(4,5-dimethylthiazol-2-yl)-2,5-diphenyltetrazolium bromide) is a tetrazolium salt that in the case of viable cells, is reduced by the strongly reducing environment of vital cell mitochondria as a result of dehydrogenase of mitochondria. The reduction of MTT leads to the formation of formazan crystals, insoluble in culture medium, but soluble in DMSO, which produces the typical purple colour of the mitochondria of vital cells. In contrast, in dead or suffering cells lacking active mitochondria, MTT will not be reduced, resulting in a less intense purple colour (Mosman, 1983). For the direct relationship between respiration and vitality, MTT is considered a good test to evaluate the viability of cells.

**.** This cytotoxicity test was used as a preliminary analysis to determine the non-cytotoxic concentrations of the test product, to derive the appropriate concentration range to be tested in the evaluation of IL-8 expression. Briefly, Hacat cells were evenly sown in 96-well plates and treated with the product from the concentration of 10 mg/ml and dilutions in series 1:2. An incubation was conducted for 24 hours. After the incubation time, a solution of MTT was added to the medium in each well for 2 hours at 37 °C. The culture medium was then removed and the cells washed with PBS and lysed with DMSO. Later, absorbance was read at 570 nm using a microplate reader. Cell cytotoxicity was calculated by measuring the percentage difference in optical density of each of the eight concentrations tested in comparison with untreated cells.

**.** The various compositions were evaluated in keratinocytes (Hacat cells) stimulated with LPS for 24 hours and treated with the products to be tested, measuring the expression of IL-8 using an ELISA kit (Ebioscience).

**.** To perform the test, Hacat cells were sown in a 96-well plate and treated with the combinations at the concentration determined by the MTT test; at the same time, the cells were stimulated with LPS. At the end of the treatment, the supernatant was collected and used to cover a specially pre-treated 96-well ELISA plate provided by the kit. The standards were reconstituted with ultra-pure water and used to build the standard curve. Samples were added to each duplicate well and the test was conducted according to the instructions for use. The absorbance was then read at 450 nm using a microplate reader (Multiskan, Thermo Fischer).

. Hacat cells were incubated (24 hours) with different concentrations of the product to identify concentrations that did not cause a decrease of more than 20% in cellular respiration (cell viability index).

### Comparative example 1 - 20 % dilution

| | | | | | | |
|---|---|---|---|---|---|---|
| Sample mg/ml | 0 | 50 | 75 | 100 | 150 | 200 |
| Cell viability (%) | 100 | 100,34 | 101,79 | 100,44 | 95,43 | 98,14 |
| Std. dev. | 0 | 5,53 | 4,26 | 1,21 | 2,79 | 3,99 |

### Comparative example 1 - 40 % dilution

| | | | | | | |
|---|---|---|---|---|---|---|
| Sample mg/ml | 0 | 50 | 75 | 100 | 150 | 200 |
| Cell viability (%) | 100 | 112,52 | 103,59 | 106,58 | 94,44 | 98,49 |
| Std. dev. | 0 | 5,32 | 8,23 | 7,02 | 8,82 | 8,73 |

### Comparative example 2 - 60% dilution

| | | | | | | |
|---|---|---|---|---|---|---|
| Sample mg/ml | 0 | 50 | 75 | 100 | 150 | 200 |
| Cell viability (%) | 100 | 93,79 | 91,98 | 90,13 | 87,05 | 80,32 |
| Std. dev. | 0 | 1,18 | 2,03 | 0,73 | 2,47 | 1,89 |

### Example 3

| | | | | | | |
|---|---|---|---|---|---|---|
| Sample mg/ml | 0 | 50 | 75 | 100 | 150 | 200 |
| Cell viability (%) | 100 | 122,33 | 124,94 | 115,13 | 107,02 | 94,15 |
| Std. dev. | 0 | 2,58 | 2,21 | 3,45 | 1,20 | 5,48 |

### Example 4

| | | | | | | |
|---|---|---|---|---|---|---|
| Sample mg/ml | 0 | 50 | 75 | 100 | 150 | 200 |
| Cell viability (%) | 100 | 134,38 | 121,08 | 114,86 | 108,30 | 99,20 |
| Std. dev. | 0 | 10,85 | 9,72 | 6,00 | 4,58 | 5,70 |

**.** The selected concentration of the product was 75 mg/ml. In fact, although the cell viability at all concentrations tested is higher than 80%, the highest concentration with the best solubility has been chosen, avoiding possible interference with the ELISA test. The possible anti-inflammatory activity of products tested after stimulation with LPS was evaluated in Hacat cells, measuring the expression of IL-8 with an ELISA kit. The positive control is represented by untreated and stimulated cells with LPS. Table 1 shows the effect on the production of IL-8, after 24 h of treatment with the compositions of examples 1-4, according to the statistical analysis performed with the method of t-Student (n=2; replicated=3).

**Table 1**

| Sample | IL-8 (%) | Variation % |
|---|---|---|
| Ctrl + LPS | 100 | - |
| Comparative example 1 | 86,89 ± 5,01 | -13,11 |
| Comparative example 2 | 90,71 ± 0,97 | -9,29 |
| Example 3 | 8,05 ± 0,15 | -91,95 |
| Example 4 | 25,67 ± 0,28 | -91,43 |

**.** The product of Examples 3 and 4, containing an oligomeric fraction, shows a clear effect on the release of interleukin IL-8, following an inflammatory stimulus (LPS) leading to an evident reduction in cytokine levels, after 24 hours of treatment; These data are highly significant, compared to the stimulated controls, (Ctrl + LPS). In contrast, the products of comparative examples 1 and 2 show a lower reduction in inflammatory marker levels than the two formulations containing the oligomeric fraction.

**.** Therefore, these results indicate an anti-inflammatory activity of the composition including the oligomer of hyaluronic acid, with a highly significant reduction of IL-8 levels after 24 hours of treatment.

## Claims

1. A process for the preparation of a HA composition having high anti-inflammatory properties, the process comprising the following steps:
a. preparing a solution of HA having molecular weight Mw comprised between 500 kDa and 6,000 kDa at a concentration comprised between 0.1 g/L and 5 g/L;
b. maintaining the temperature of the solution lower than or equal to 10°C;
c. submitting the solution to pulse sonication for a time comprised between 1' and 30', wherein the pulse of pulse sonication is comprised between 5" and 15" for both the sonication phase and the pause between two sonications, and the sonication power per mass unit of solution is from 200 to 2000 kW/kg.

2. The process according to claim 1, further comprising the step:
d. mixing sonicated HA of step c) with at least one high Mw HA having molecular weight Mw comprised between 500 kDa and 6,000 kDa.

3. The process of claim 2, wherein the weight ratio between sonicated HA and the sum of sonicated HA and high Mw HA is comprised between 5 % and 100%.

4. The process of claim 3, wherein the ratio is comprised between 10% and 50%.

## Patentansprüche

1. Verfahren zur Herstellung einer HA-Zusammensetzung mit hohen entzündungshemmenden Eigenschaften, wobei das Verfahren die folgenden Schritte umfasst:
a. Herstellen einer HA-Lösung mit einem Molekulargewicht Mw zwischen 500 kDa und 6.000 kDa bei einer Konzentration zwischen 0,1 g/L und 5 g/L;
b. Aufrechterhalten der Temperatur der Lösung bei kleiner oder gleich 10°C;
c. Unterwerfen der Lösung einer gepulsten Sonikation für eine Zeit zwischen 1' und 30', wobei der Puls der gepulsten Sonikation sowohl für die Sonikationsphase als auch für die Pause zwischen zwei Sonikationen zwischen 5" und 15" liegt und die Sonikationsleistung pro Masseneinheit der Lösung zwischen 200 und 2000 kW/kg beträgt.

2. Verfahren nach Anspruch 1, ferner umfassend den Schritt:
d. Mischen des sonifizierten HA aus Schritt c) mit mindestens einem hochmolekularen HA mit einem Molekulargewicht Mw zwischen 500 kDa und 6.000 kDa.

3. Verfahren nach Anspruch 2, wobei das Gewichtsverhältnis zwischen sonifiziertem HA und der Summe aus sonifiziertem HA und hochmolekularem HA zwischen 5 % und 100 % liegt.

4. Verfahren nach Anspruch 3, wobei das Verhältnis zwischen 10 % und 50 % liegt.

## Revendications

1. Procédé pour la préparation d'une composition de HA ayant des propriétés antiinflammatoires élevées, le procédé comprenant les étapes suivantes :
a. préparer une solution de HA ayant un poids moléculaire Mw compris entre 500 kDa et 6 000 kDa à une concentration comprise entre 0,1 g/L et 5 g/L ;
b. maintenir la température de la solution inférieure ou égale à 10°C ;
c. soumettre la solution à une sonication pulsée pendant un temps compris entre 1' et 30', dans lequel l'impulsion de la sonication pulsée est comprise entre 5" et 15" tant pour la phase de sonication que pour la pause entre deux sonications, et la puissance de sonication par unité de masse de solution est comprise entre 200 et 2000 kW/kg.

2. Procédé selon la revendication 1, comprenant en outre l'étape :
d. mélanger le HA soniqué de l'étape c) avec au moins un HA de haut Mw ayant un poids moléculaire Mw compris entre 500 kDa et 6 000 kDa.

3. Procédé selon la revendication 2, dans lequel le rapport pondéral entre le HA soniqué et la somme du HA soniqué et du HA de haut Mw est compris entre 5 % et 100 %.

4. Procédé selon la revendication 3, dans lequel le rapport est compris entre 10 % et 50 %.
